(19) Europäisches Patentamt
European Patent Office
Office européen des brevets

(11) Publication number: 0 161 043
A2

# (12) EUROPEAN PATENT APPLICATION

(21) Application number: 85301431.4

(22) Date of filing: 01.03.85

(51) Int. Cl.[4]: C 08 G 18/64
C 12 N 11/08, C 08 G 18/14

(30) Priority: 02.03.84 US 585744
25.10.84 US 664664

(43) Date of publication of application:
13.11.85 Bulletin 85/46

(84) Designated Contracting States:
BE CH DE FR GB IT LI NL SE

(71) Applicant: THE DOW CHEMICAL COMPANY
2030 Dow Center Abbott Road P.O. Box 1967
Midland, MI 48640(US)

(72) Inventor: Freedman, Harold H.
141 Jackson Street
Newton Center Massachusetts 02159(US)

(74) Representative: Allard, Susan Joyce et al,
BOULT, WADE & TENNANT 27 Furnival street
London EC4A 1PQ(GB)

## (54) Process for preparation of crosslinked polyamines and crosslinked gelatin foams.

(57) Polyalkylenepolyamines and gelatins are crosslinked in a non-anhydrous anvironment to yield water-swellable, essentially water-insoluble gels and foams. The polyalkylenepolyamine is contacted with a polyisocyanate at a pH between 5 and 8 and subjected to a high rate of agitation. The gelatin is contacted with a polyisocyanate at a pH between 6 and 8 and subjected to a high rate of agitation. The process of this invention can be employed to immobilize proteins, enzymes, antibodies, etc.

EP 0 161 043 A2

Croydon Printing Company Ltd

0161043

# PROCESS FOR PREPARATION OF CROSSLINKED POLYAMINES AND CROSSLINKED GELATION FOAMS

This invention relates to crosslinked polymers, and in particular, to crosslinked polymers which form water-swellable, water-insoluble gels and water-swellable, water-insoluble foams.

Linear polyalkylenepolyamines are rendered water-insoluble through the addition of diisocyanates at a temperature between 30°C and 200°C, using techniques disclosed in U.S. 4,087,413. Unfortunately, the resulting water-insoluble products are prepared either in neat form or in the presence of a suitable solvent. Reactions of amines with diisocyanates are disclosed in U.S. 4,177,038. However, it is typically necessary to prepare such products under essentially anhydrous conditions because isocyanates moieties are hydrolyzed rapidly by water to yield substituted ureas.

In view of the fact that polymers such as acylated polyalkylenepolyamines are water-soluble, and in view of the fact that it would be highly desirable to render such polymers water-insoluble in an aqueous medium; it would be highly desirable to provide a

process for preparing a crosslinked polyalkenepolyamine in a non-anhydrous environment.

In view of the fact that known processes provide gelled materials, it would also be highly desirable to provide a process for efficiently preparing stable, water-swellable but water-insoluble foams.

The present invention is a process for crosslinking a polyalkylenepolyamine wherein a polyisocyanate is contacted with an aqueous liquid comprising a polyalkylenepolyamine which is at a specific pH and which is subjected to a sufficiently high rate of shear agitation to yield a water-swellable, essentially water-insoluble gel. By the term "specific pH" is meant a pH which can range from slightly acidic to slightly basic.

The advantage of the process of this invention is that gelled materials can be prepared quickly and efficiently under conditions which do not require an anhydrous environment. The gels are useful in a wide variety of applications in which water-insoluble, swellable gels are useful. Of particular interest, are those uses in which a gel is useful as a biosupport for the isolation and purification of enzymes and other proteins.

Thus, in another aspect the present invention is a process for covalently immobilizing a protein wherein said protein is contacted with a polyalkylene-polyamine and a polyisocyanate in an aqueous liquid which is at a specific pH and which is subjected to a sufficiently high rate of agitation to yield a water-swellable, essentially water-insoluble gel.

Still another aspect, the present invention is a process for providing stable, water-swellable, essentially water-insoluble foam wherein a polyisocyanate is contacted with an aqueous liquid comprising a gelatin which is at a specific pH and which is subjected to a sufficiently high rate of shear agitation. By the term "specific pH" is meant a pH which can range from slightly acidic to slightly basic.

Thus, another advantage of the process of this invention is that foamed materials can be prepared quickly and efficiently under conditions which do not require an anhydrous environment. Foams which are prepared can be dried in order to provide gels which are hard, soft, friable, etc. Upon drying the foam structure is maintained and the original gel can be regenerated by swelling in water. Typically, foams are closed cell foams. The foams are useful in a wide variety of applications in which water-insoluble, swellable foams are useful. Of particular interest, are those uses in which a foam is useful as a biosupport for the isolation and purification of enzymes and other proteins where a proteinaceous environment is desirable.

Thus, still in another aspect the present invention is a process for covalently immobilizing a protein wherein said protein is contacted with a gelatin and a polyisocyanate in an aqueous liquid which is at a specific pH and which is subjected to a sufficiently high rate of agitation to yield a water-swellable, essentially water-insoluble foam.

The immobilized proteins are useful for a variety of uses. For example, immobilized proteins can be used as catalysts or in the purification of bioproducts.

As used herein, the term "gelatin" refers to those types of proteins which are obtained by the hydrolysis of collagen by boiling skin, ligaments, tendons, etc. Type A (obtained from acid treated raw materials) and Type B (obtained from alkali treated raw materials) can be employed. Most grades of commercially available purified gelatins can be employed. Gelatins having a high bloom number; for example, greater than about 100; are preferred.

Polyalkylenepolyamines have repeating units which can be independently represented as follows:

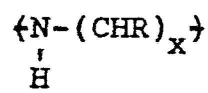 , (I)

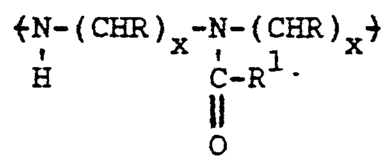 , (II)

or 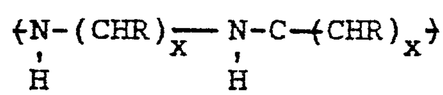 , (III)

wherein R is independently hydrogen or a $C_1$ to $C_3$ lower alkyl; $R^1$ is $C_1$ to $C_{18}$ alkyl, aryl, alkylaryl or substituted alkyl, alkylaryl or aryl; and x is 2 or 3. Such types of polymers are prepared using techniques disclosed in U.S. 4,087,413. In general, the polyalkylenepolyamines of this invention are any water-soluble

polyalkylenepolyamines containing secondary amine groups. The preferred polyalkylenepolyamine is polyethylenepolyimine. In the case of the partially hydrolyzed acylated polyamines, the extent of hydrolysis can range from 10 to 85, preferably 50 weight percent, based on the weight of the polymerized monomers. Preferably, the molecular weight of the polyalkylenepolyamine can range from 10,000 to 1,000,000; most preferably from 50,000 to 75,000. Poor gels are obtained or no gelation occurs if the molecular weight of the polymer is not sufficiently high.

Organic polyisocyanates which can be employed include aromatic, aliphatic and cycloaliphatic polyisocyanates and combinations thereof. Representative of these types are the diisocyanates such as m-phenylene diisocyanate, tolylene-2,4-diisocyanate, tolulene-2,6-diisocyanate, hexamethylene-1,6-diisocyanate, tetramethylene-1,4-diisocyanate, cyclohexane-1,4-diisocyanate, hexahydrotolylene diisocyanate (and isomers), naphthylene-1,5-diisocyanate, 1-methoxyphenyl-2,4-diisocyanate, diphenylmethane-4,4-diisocyanate, 4,4'-biphenylene diisocyanate, 3,3'-dimethoxy-4,4'-biphenyl diisocyanate, 3,3'-dimethyl-4,4'-biphenyl diisocyanate, and 3,3'-dimethyldiphenylmethane-4,4'-diisocyanate; the triisocyanates such as 4,4',4'-triphenylmethane triisocyanate, polymethylene polyphenylisocyanate and tolylene-2,4,6-triisocyanate; and the tetraisocyanates such as 4,4'-dimethyldiphenylmethane-2,2',5,5'-tetraisocyanate. Especially useful due to their availability and properties are tolulene diisocyanate, diphenylmethane-4,4'-diisocyanate and polymethylene polyphenylisocyanate.

Crude polyisocyanate can also be used in the practice of the present invention, such as crude toluene diisocyanate obtained by the phosgenation of a mixture of toluene diamines or crude diphenylmethylene diisocyanate obtained by the phosgenation of crude diphenylmethylenediamine. The preferred undistilled or crude isocyanates are disclosed in U.S. 3,215,652.

The amount of polyisocyanate which is employed relative to the polyalkylenepolyamine can vary depending upon factors such as the reactivity of the species, the concentration of reactants relative to the aqueous liquid, the amount of crosslinking desired and the like. Preferably, the amount of polyisocyanate ranges from 5 to 25, preferably 10 to 15 weight percent, based on the weight of the dry polyalkylenepolyamine. Typically, poor gels are obtained or no gelation occurs if there is employed an insufficient amount of polyisocyanate.

The amount of polyisocyanate which is employed relative to the gelatin can vary depending upon factors such as the reactivity of the species, the concentration of reactants relative to the aqueous liquid, the amount of crosslinking desired and the like. Preferably, the amount of polyisocyanate ranges from 1 to 20, preferably 2 to 8 weight percent, based on the weight of the dry gelatin. Typically, poor foams are obtained or no permanent foaming occurs if there is employed an insufficient amount of polyisocyanate.

The process of this invention is perfomed in an aqueous liquid. Preferably, the aqueous liquid comprises essentially water. As used herein, the term "aqueous liquid" means a liquid comprising water which

can contain additives soluble therein or immiscible therewith. For example, the aqueous liquid can contain an aqueous phase comprising water and additives soluble therein, and a water immiscible organic solvent. The amount of the water immiscible organic solvent can range, for example, from 0.5:1 to 5:1 relative to the volume of the aqueous phase. Examples of water immiscible organic solvents include benzene, toluene, methylenechloride, chloroform, and the like.

Typically, depending upon the polyamine, the pH of the aqueous liquid can range from 4 to 9, preferably 5 to 8, more preferably from 5 to 7.5, most preferably from 6 to 6.5. Also, depending upon gelation, the pH of the aqueous liquid can range from 4 to 10, preferably 6 to 8. The pH of the aqueous liquid is critical as the rate of crosslinking is sensitive to pH. That is, for example, the crosslinking reaction can occur too quickly if the pH is exceedingly low. Conversely, the crosslinking reaction can occur very slowly if the pH is exceedingly high. In addition, poor gels and foams are obtained or no gelation or foaming occurs if the pH of the aqueous liquid is not within a suitable (i.e., specific) range. The pH typically decreases by a small amount (i.e., less than 1 unit) during the crosslinking reaction. This change in pH can be controlled by a suitable buffer.

The amount of aqueous liquid which is employed depends upon the molecular weight and the amount of polyalkylenepolyamine or gelatin which is employed. Typically, poor gelation or foaming occurs if the polymer solution is very dilute or the molecular weight of the polymer is very low. Conversely, the formation

of desirable gels or foams is a difficult process to perform if the polymer concentration in the aqueous liquid is very high or the molecular weight of the polymer is very high. Preferably, the amount of polyalkylenepolyamine present in the aqueous liquid can range from 10 to 50, preferably from 20 to 30 weight percent, based on the weight of the polymer and aqueous liquid. Preferably, the amount of gelatin present in the aqueous liquid can range from 0.5 to 20, preferably from 1 to 10 weight percent, based on the weight of the polymer and aqueous liquid.

The temperature at which the process of this invention is performed is not particularly critical and can vary. For example, the process of this invention can be performed from 0° to 30°C. Typically, crosslinking can occur at room temperature. However, the aqueous liquid can also be heated above 30°C.

The process of this invention is highly dependent upon the rate of agitation to which the aqueous polyalkyleneamine/polyisocyanate or gelatin/-polyisocyanate mixture is subjected. Little or no gellation or foaming occurs when a conventional magnetic stirrer or mechanical stirrer is employed as an agitation device. For this reason, it is necessary to provide a sufficiently high rate of agitation using a device such as a homogenizer, ultra-sonic stirring device or blender. Typically, the agitation rate should exceed 500 rpm, preferably 3,000 rpm for formation process. Typically, the agitation rate should exceed 3,000 rpm for the gelation process. Preferred agitation rates range from 5,000 rpm to 50,000 rpm, most preferably from 10,000 rpm to 20,000 rpm. It is understood that practically

any device capable of providing such a high rate of agitation can be effectively employed.

The polyalkyleneamine/polyisocyanate or gelative/polyisocyanate mixture is subjected to a high rate of agitation for a period of time which can vary. For example, the period of time over which the mixture is subjected to agitation can depend upon factors such as the amount of reactants, the concentration of reactants in the aqueous liquid, etc. Typically, the period of time over which the polyalkyleneamine/polyisocyanate mixture is subjected to agitation can range from 2 seconds to several seconds, most preferably from 3 seconds to 5 seconds. Typically, the period of time over which the gelatin/polyisocyanate mixture is subjected to agitation can range from 2 seconds to several minutes, preferably from 20 seconds to 2 minutes, most preferably for 30 seconds.

In the situation in which a two phase (i.e., aqueous solvent and water-immiscible solvent) solvent system is employed, hydrolysis of the isocyanate is believed to be retarded. Thus, conventional stirring devices such as magnetic stirrers or mechanical stirrers can be employed to yield good results. However, the rate of crosslinking is much slower than that observed when high rates of agitation are employed. That is, reaction times ranging from several minutes to 1 hour may be necessary for sufficient crosslinking to occur. In any event, the actual reaction time can depend upon factors such as the particular polymer, the isocyanate concentration, the relative amount of solvent, the proportions of aqueous phase and water immiscible phase, etc.

After the mixture is subjected to agitation, the mixture is allowed to settle for a period of time sufficient for gellation or foams formulation (i.e., crosslinking) to be completed. This period of time ranges from 10 minutes to 60 minutes, preferably from 15 to 20 minutes for gelation. This period of time ranges from a few minutes to several hours, preferably from 1 hour to 8 hours, most preferably for 2 hours for foam formulations. Setting can occur over a wide temperature range, but preferably occurs at room temperature.

The process of this invention is most preferably carried out in a predominately aqueous phase. It is understood that most additives common in an aqueous liquid can be present. For convenience purposes, the water-soluble polyalkylenepolyamine or gelatin is dispersed in the aqueous liquid and a solution is formed, which solution has the appropriate pH. This solution is contacted with the polyisocyanate, and the mixture is subjected to a sufficiently high rate of agitiation for a short period of time. The mixture is then allowed to gel or set as appropriate. If desired, the resulting gel or foam can be dispersed in an aqueous liquid by providing further agitation. If desired, the foam can be dried. In any case, the gel or foam can be further treated, as desired, for the particular application for which it is employed.

Proteins containing free primary amine groups, such as enzymes and antibodies, can be immobilized using the process of this invention. For example, glucose oxidase, esterase, catalase, alkaline phosphatase, and the like can be easily and effectively immobilized.

Preferably, the polyalkylenepolyamine or gelatin, polyisocyanate and protein are contacted in the suitable solution at the suitable pH and subjected to the appropriate agitation. Typically, the amount of protein which is employed ranges from 0.1 to 10 milligrams per milliliter of polyalkylenepolyamine or gelatin which is employed.

When employed as a biosupport for enzyme immobilization, the reaction conditions under which the gels are formed can be modified in accordance with the requirements necessary for the individual enzyme. Covalent attachment of the enzyme to the gel or foam is believed to occur during the mixing and gellation or foamation stages. It is believed that immobilization occurs via the reaction of free isocyanate moieties of the gel or foam with amino moieties of the protein. If desired, the enzyme active site can be protected from reaction by the presence of suitable enzyme substrates and/or products. The amount of immobilized protein can vary. The enzyme activity of the immobilized enzyme can also vary and is typically from 20 to 80 percent of its original value.

The gels which are prepared are very hydrophilic, though essentially water-insoluble. When dry, the gels are amorphous, hard, solid materials which can be easily handled. When fully hydrated, the gels are transparent to translucent, soft, compressible solids which are insoluble in water.

The foams which are prepared are very hydrophilic, though essentially water-insoluble. When dry, the foams can be hard and/or friable solid materials

which maintain foam structure and can be easily handled. When fully hydrated, the foams are transparent to transucent, soft, compressible solids which are insoluble in water. The foams are typically closed cell foams.

The gels or foams can be employed as water adsorbent materials, insoluble chelants for metals, insoluble modifiers of pH for aqueous systems, biosupports, and other such uses.

The following examples are presented to further illustrate the scope of this invention.

Example 1

A 50 percent aqueous solution of polymer was prepared by dispersing 5 parts by weight of a high molecular weight polyethyleneimine in water. The pH of the solution was adjusted to 6.5 using concentrated hydrochloric acid. The solution was diluted with water to yield a solution containing 30 percent polyethyleneimine. To this solution was added 0.2 parts by weight of hexamethylene-diisocyanate. The mixture was homogenized using a blender (agitation rate was about 10,000 rpm) for 5 seconds. The mixture was allowed to stand for 20 minutes. The resulting firm gel was broken into pieces, stirred with 100 ml water and centrifuged for 5 minutes. The supernatant liquid was decanted. The resulting gel has a volume of about 50 ml at a pH of about 5.

Example 2

A 25 percent aqueous solution of polymer was prepared by dispersing 5 g of said polymer in water in an amount sufficient to provide a 20 ml aqueous solution.

The polymer was a 50 percent hydrolyzed polyethyloxazoline having a molecular weight of 50,000. The pH of the solution was adjusted to 8 using hydrochloric acid. The solution was magnetically stirred in a beaker and 15 ml of methylene chloride and 0.4 ml of 1,6-hexamethylene diisocyanate was added thereto. The mixture was rapidly stirred for 15 minutes using a magnetic stirrer set at the highest obtainable speed (estimated at 200 rpm). The mixture was allowed to stand for 1 hour. The product was triturated 3 times with 40 ml each of acetone. The resulting white, acetone-swollen gel was dried to give 4 g of white granular solid.

Example 3

The immobilization of glucose oxidase (GOD) using polyethyleneimine was performed as follows.

To 1 ml of an aqueous polyethylimine solution (obtained from Aceto Chemicals, under the specification PEI-1,000) and adjusted to pH 6.1 and concentration of 0.25 g/ml was added 1 ml of aqueous solution containing 50 units (U) GOD and 20 μl of 1,6,-hexamethylene dissocyanate and the mixture was homogenized for 5 seconds using a laboratory model Ross homogenizer at its maximum speed. The gel was allowed to sit for 15 minutes, diluted to 20 ml with water and dispersed by stirring at low speed in a 3-speed Waring Blender. The total gel, (20 ml) which assayed for 35 U was centrifuged and the supernatant (6 ml) was decanted. The supernatant assayed for 4 U and the washed gel containing the immobilized GOD assayed for 33 U. After sitting at room temperature for 1 week, the gel assayed for 38 U.

Example 4

The immobilization of esterase using hydrolyzed polyethyloxazoline was performed as follows.

To 5 ml of a 15 percent solution of 50 percent hydrolyzed polyethyloxazoline (M.W. = 50 M, pH 7.6) was added 100 µl of esterase (about 100 U) and 200 µl 1,6-hexamethylene dissocyanate and the mixture was homogenized for 10 seconds on a Vertis Model 45 homogenizer set at medium speed and left for 1 hour. The firm gel was chopped up in a Waring Blender with 10 ml of water, made up to 20 ml, stirred for 30 minutes and then centrifuged. The supernatant had no activity and the gel assayed for 50 U. The yield of immobilized esterase is 50 percent.

Example 5

15 ml of a 7.5 percent solution of 300 bloom swine skin gelatin was adjusted to a pH of 8. To this mixture was added 0.1 ml hexamethylene-2,4-diisocyanate and the mixture was homogenized for 30 seconds at high speed using a Ross Laboratory Homogenizer (12 mm head). The resulting foam which has a volume of about 25 ml was set in about one minute. The foam was air dried to yield about 15 ml of a firm white foam.

Example 6

15 ml of a 7.5 percent solution of 200 bloom technical grade gelatin containing 0.15 ml of alkaline phosphatase (Sigma No. P4502) was adjusted to a pH of 8. To this mixture was added 0.1 ml hexamethylene-2,4-diisocyanate and the mixture was homogenized as in Example 5. The foam gels slowly enough to be cast as a film. After air drying for three days and reswelling

in water, the washed film assays for most of the original enzyme activity.

Example 7

5 g of a 10 percent solution of 200 bloom gelatin was diluted with 10 ml water and the pH of the mixture was adjusted to about 8.5. To this was added one drop of a liquid emulsifier. To this was added 0.1 ml of hexamethylene-2,4-diisocyanate and the mixture was immediately homogenized as described in Example 5. The mixture sets to a white, soft foam with a volume of about 50 ml. After thorough drying at room temperature, the volume of the white collapsed foam was about 20 ml and the foam weighs 0.7 g.

CLAIMS:

1. A process for crosslinking a polyalkylenepolyamine which comprises contacting a polyisocyanate with an aqueous liquid comprising a polyalkylenepolyamine which is at a specific pH and subjecting the mixture to a sufficiently high rate of agitation to yield a water-swellable, essentially water-insoluble gel.

2. A process as claimed in Claim 1 wherein the aqueous liquid comprises essentially water.

3. A process as claimed in Claim 1 or Claim 2 wherein in the rate of agitation is in excess of 3,000 rpm.

4. A process as claimed in any one of the preceding Claims wherein the specific pH is in the range of from 5 to 8.

5. A process as claimed in Claim 1 wherein the aqueous liquid comprises water and a water immiscible organic solvent.

6. A process as claimed in any one of the preceding Claims wherein the polyalkylenepolyamine has repeating units which are independently represented as:

$$\left( \underset{H}{N} - (CHR)_x \right) \quad , \qquad (I)$$

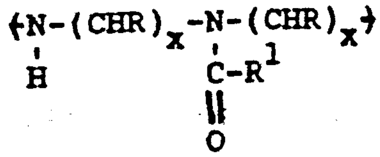 , (II)

or 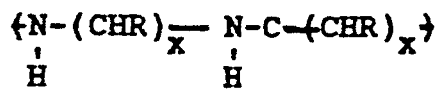 , (III)

wherein R is independently hydrogen or $C_1$ to $C_3$ lower alkyl; $R^1$ is $C_1$ to $C_{18}$ alkyl, aryl, alkylaryl or substituted alkyl, aryl or alkylaryl; and x is 2 or 3.

7. A process as claimed in Claim 6 wherein the polyalkylenepolyamine is polyethylenepolyimine.

8. A process as claimed in any one of the preceding Claims wherein the polyisocyanate is a diisocyanate.

9. A process as claimed in Claim 1 wherein the aqueous liquid contains a protein.

10. A process as claimed in Claim 9 wherein the protein is an enzyme.

11. A process for producing a stable, water-swellable, essentially water-insoluble foam which comprises contacting a polyisocyanate with an aqueous liquid comprising a gelatin which is at a specific pH and subjecting the mixture to a sufficiently high rate of shear agitation to yield the water-swellable, essentially water-insoluble foam.

12. A process as claimed in Claim 11 wherein the aqueous liquid comprises essentially water.

13. A process as claimed in Claim 11 or Claim 12 wherein the rate of agitation is in excess of about 3,000 rpm.

14. A process as claimed in any one of Claims 11 to 13 wherein the specific pH is in the range of from 6 to 8.

15. A process as claimed in Claim 11 wherein the aqueous liquid comprises water and a water immiscible organic solvent.

16. A process as claimed in any one of Claims 11 to 15 wherein the polyisocyanate is a diisocyanate.

17. A process as claimed in any one of Claims 11 to 16 wherein the aqueous liquid comprises a protein.

18. A process as claimed in Claim 17 wherein protein is an enzyme.